(19) **Europäisches Patentamt European Patent Office Office européen des brevets**

(11) **EP 3 123 994 B1**

(12) **EUROPEAN PATENT SPECIFICATION**

(45) Date of publication and mention of the grant of the patent:
**20.06.2018 Bulletin 2018/25**

(21) Application number: **15769118.9**

(22) Date of filing: **24.03.2015**

(51) Int Cl.:
*A61F 13/47* (2006.01)       *A61F 13/49* (2006.01)
*A61F 13/533* (2006.01)       *A61F 13/536* (2006.01)
*A61F 13/511* (2006.01)       *A61F 13/513* (2006.01)
*A61F 13/534* (2006.01)

(86) International application number:
**PCT/JP2015/058943**

(87) International publication number:
**WO 2015/146991 (01.10.2015 Gazette 2015/39)**

(54) **ABSORBENT PRODUCT**

SAUGFÄHIGES PRODUKT

PRODUIT ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2014 JP 2014062128**

(43) Date of publication of application:
**01.02.2017 Bulletin 2017/05**

(73) Proprietor: **Unicharm Corporation Ehime 799-0111 (JP)**

(72) Inventors:
• **TOKITA, Norihiro**
  **Kanonji-shi**
  **Kagawa 769-1602 (JP)**

• **GOHDA, Hiroki**
  **Kanonji-shi**
  **Kagawa 769-1602 (JP)**

(74) Representative: **Peter, Julian**
  **Staeger & Sperling**
  **Partnerschaftsgesellschaft mbB**
  **Sonnenstrasse 19**
  **80331 München (DE)**

(56) References cited:
| | |
|---|---|
| EP-A1- 0 769 284 | WO-A1-00/32145 |
| WO-A1-2010/107096 | WO-A1-2012/073499 |
| JP-A- 2005 034 652 | JP-A- 2011 156 032 |
| JP-A- 2012 125 354 | US-A1- 2005 124 951 |

**Description**

Technical Field

The present invention relates to a disposable diaper.

Background Art

The disposable diaper described in Japanese Unexamined Patent Publication No. 2006-141761 has slits between a center absorbent body and a pair of side absorbent bodies, the slits being provided at both ends of the center section at least in the lengthwise direction of the center absorbent body (product length direction), and being open at both ends of the other section in the lengthwise direction (the rear body section), and the side absorbent bodies being situated in at least the lower crotch region (near the center section of the product). Since this prior art example has slits, the lower crotch region is not loose when the diaper is worn, the outer appearance of the diaper is smooth, the fitting property and leakproofness are excellent at the crotch section, and production stability (continuous productivity) at the diaper production site is excellent.

Citation List

Patent Literature

[PTL 1] Japanese Unexamined Patent Publication No. 2006-141761 Further prior art in this technical field is disclosed in documents EP 0 769 284 A1, WO 00/32145 A1, US 2005/124951 A1 and WO 2012/073499A1.

DISCLOSURE OF THE INVENTION

Problems to be Solved by the Invention

With a diaper provided with slits in the absorbent body, the degree of freedom of deformation is too high when the diaper is worn, and therefore the absorbent body undergoes undesirable deformation, resulting in problems such as entering of the diaper between the buttocks. In addition, when used for long periods (during nighttime or when outdoors) or when worn by infants or children that experience intense movement, this has created a load on the absorbent body either before or after absorption, and the shape of the absorbent body has sometimes collapsed. Furthermore, when the density is overly increased by, for example, excessive pressing of the absorbent body, in order to minimize such collapse, it has become impossible to maintain the softness of the diaper.
It is an object of the present invention to provide an absorbent article wherein the absorbent body section easily deforms to the intended shape, and collapse of the shape of the absorbent body during use can be prevented.

Means for Solving the Problems

The present inventors have completed this invention upon finding that the problems associated with the example of the prior art described above can be solved by providing the absorbent body of the absorbent article with fold-inducing bands having low basis weight and a low super-absorbent polymer content, further providing it with high density compression grooves in the fold-inducing bands, and situating the fold-inducing bands up to a location near the absorbent body side edges.
Specifically, the present invention is a diaper having the features of claim 1 and comprising a liquid-permeable top sheet, a liquid-impermeable back sheet, an absorbent body lying between them, a pair of left and right side sheets forming leak barriers, and an outer sheet,
the absorbent body including hydrophilic fibers and a super-absorbent polymer,
each side sheet consisting of a front edge region, a center region and a rear edge region, a widthwise outside region of the side sheet being joined with at least one of the top sheet, back sheet and outer sheet, the side sheet being joined with the top sheet at widthwise inside edge sections of the front edge region and the rear edge region of the side sheet, a widthwise inside edge of the center region of the side sheet being a free edge, wherein an elastic member is mounted at the free edge and the center region of the side sheet functions as a leak barrier,
the absorbent body consisting of a front region, a crotch region and a back region, there being provided in the absorbent body a pair of left and right fold-inducing bands extending both forward and backward from a center of the crotch region, a distance between the right fold-inducing band and the left fold-inducing band in the widthwise direction being wider at front edges and rear edges of the fold-inducing bands compared to the center of the crotch region, the front edges and rear edges of the fold-inducing bands being located more toward an outer side in a widthwise direction than widthwise inside locations of the joined areas between the side sheets and the top sheet at the widthwise inside edge sections of the front edge regions and rear edge regions of the side sheets, the fold-inducing bands including hydrophilic fibers and a super-absorbent polymer, but having a lower basis weight and a lower super-absorbent polymer content than the other sections of the absorbent body,

wherein a compression groove is formed in each fold-inducing band, the compression groove having higher density than the other sections of the absorbent body.

The invention encompasses the following modes.

[1] An absorbent article comprising a liquid-permeable top sheet, a liquid-impermeable back sheet, an absorbent body lying between them, a pair of left and right side sheets forming leak barriers, and an outer sheet,

the absorbent body including hydrophilic fibers and a super-absorbent polymer,
each side sheet consisting of a front edge region, a center region and a rear edge region, a widthwise outside region of the side sheet being joined with at least one of the top sheet, back sheet and outer sheet, the side sheet being joined with the top sheet at widthwise inside edge sections of the front edge region and the rear edge region of the side sheet, a widthwise inside edge of the center region of the side sheet being a free edge, wherein an elastic member is attached at the free edge and the center region of the side sheet functions as a leak barrier, and
the absorbent body consisting of a front region, a crotch region and a back region, there being provided in the absorbent body a pair of left and right fold-inducing bands extending both forward and backward from a center of the crotch region, a distance between the right fold-inducing band and the left fold-inducing band in the widthwise direction being wider at front edges and rear edges of the fold-inducing bands compared to the center of the crotch region, the front edges and rear edges of the fold-inducing bands being located more toward an outer side in a widthwise direction than widthwise inside locations of the joined areas between the side sheets and the top sheet at the widthwise inside edge sections of the front edge regions and rear edge regions of the side sheets, and the fold-inducing bands including hydrophilic fibers and a super-absorbent polymer, but having a lower basis weight and a lower super-absorbent polymer content than the other sections of the absorbent body,

wherein a compression groove is formed in each fold-inducing band, the compression groove having higher density than the other sections of the absorbent body.

The front edges and rear edges of the fold-inducing bands are located on the side edges of the absorbent body.

The fold-inducing bands have curved or broken line shapes that are convex toward the widthwise center of the absorbent body.

The basis weight of the absorbent body is lower in the regions more outward in the widthwise direction than the fold-inducing bands, compared to the regions more inward in the widthwise direction than the fold-inducing bands. The basis weight of the absorbent body in the fold-inducing bands is 35 to 65 % by weight of the basis weight of the absorbent body in the regions more inward in the widthwise direction than the fold-inducing bands. The basis weight of the hydrophilic fibers in the fold-inducing bands is 45 to 80 % by weight of the basis weight of the hydrophilic fibers in the regions more inward in the widthwise direction than the fold-inducing bands, and the basis weight of the super-absorbent polymer in the fold-inducing bands is 25 to 50 % by weight of the basis weight of the super-absorbent polymer in the regions more inward in the widthwise direction than the fold-inducing bands. The basis weight of the absorbent body in the regions more outward in the widthwise direction than the fold-inducing bands is 50 to 80 % by weight of the basis weight of the absorbent body in the regions more inward in the widthwise direction than the fold-inducing bands.

Effect of the Invention

The diaper of the invention has an absorbent body section that easily deforms to the intended shape and can help prevent collapse of the shape of the absorbent body during use, while it is also resistant to leakage.

Brief Description of the Drawings

Fig. 1 is a plan view showing one embodiment of the absorbent article of the invention.
Fig. 2 is a cross-sectional view along line I-I of Fig. 1.
Fig. 3 shows the shape of the fold-inducing band according to another embodiment.
Fig. 4 is a diagram showing an example of a production apparatus for an absorbent body.
Fig. 5 is a diagram showing an example of a cross-section of the layering section of a first layering drum.
Fig. 6 is a diagram showing another example of a cross-section of the layering section of a first layering drum.

Fig. 7 is a diagram showing an example of a cross-section of the layering section of a second layering drum.
Fig. 8 is a diagram showing an example of the embossing shape of a pressured embossing roll.

Best Mode for Carrying Out the Invention

The invention will now be described with reference to the accompanying drawings, with the understanding that the invention is not limited to the depictions in the drawings.

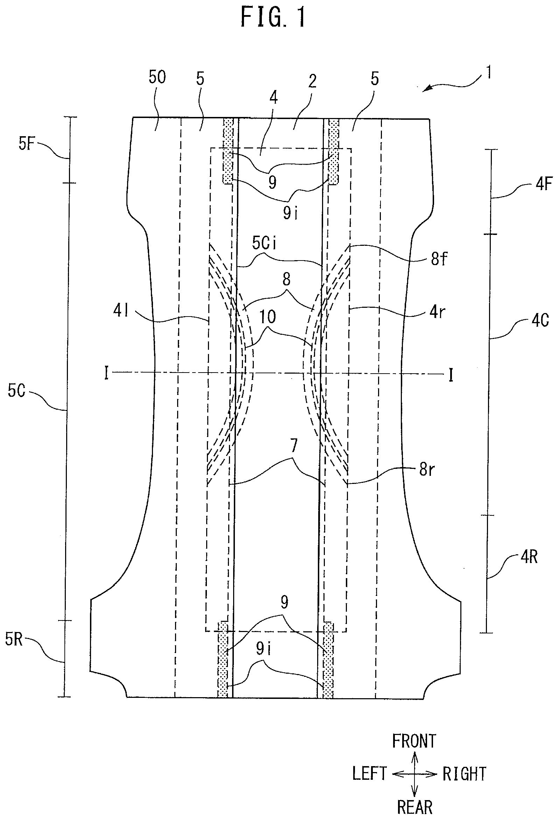

Fig. 1 is a plan view showing an embodiment of an absorbent article according to the invention. The absorbent article of Fig. 1 is an example of a disposable diaper.

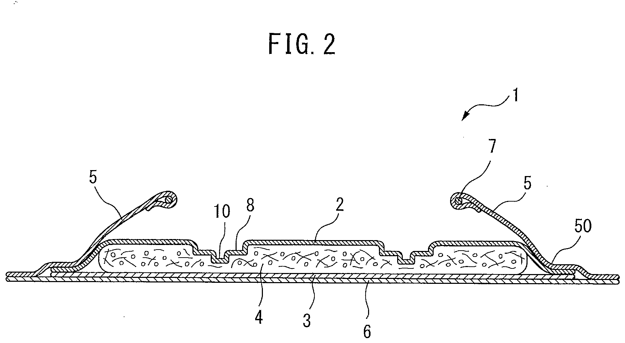

Fig. 2 is a cross-sectional view along line I-I of Fig. 1.

The absorbent article 1 of the invention comprises a liquid-permeable top sheet 2, a liquid-impermeable back sheet 3, an absorbent body lying between them 4, a pair of left and right side sheets 5, 5 forming leak barriers, and an outer sheet 6.

The absorbent body 4 includes hydrophilic fibers and a super-absorbent polymer.

Each side sheet 5 consists of a front edge region 5F, a center region 5C and a rear edge region 5R, the widthwise outside region 5O of the side sheet being joined with at least one of the top sheet 2, back sheet 3 and outer sheet 6, the side sheet 5 being joined with the top sheet 2 at the widthwise inside edge sections of the front edge region and the rear edge region of the side sheet, the widthwise inside edge of the center region 5Ci of the side sheet being a free edge, wherein an elastic member 7 is attached at the free edge and the center region of the side sheet functions as a leak barrier.

The absorbent body 4 consists of a front region 4F, a crotch region 4C and a back region 4R, there being provided in the absorbent body 4 a pair of left and right fold-inducing bands 8, 8 extending both forward and backward from the center of the crotch region, the distance between the right fold-inducing band and the left fold-inducing band in the widthwise direction being wider at the front edges 8f and rear edges 8r of the fold-inducing bands compared to the center of the crotch region, the front edges 8f and rear edges 8r of the fold-inducing bands being located more toward the outer side in the widthwise direction than the widthwise inside locations 9i of the joined areas 9 between the side sheets and the top sheet at the widthwise inside edge sections of the front edge regions and rear edge regions of the side sheets, the fold-inducing bands 8 including hydrophilic fibers and a super-absorbent polymer, but having a lower basis weight and a lower super-absorbent polymer content than the other sections of the absorbent body.

A compression groove 10 is provided in each fold-inducing band 8, the compression groove 10 having higher density than the other sections of the absorbent body.

The fold-inducing band 8 is a region that includes hydrophilic fibers and a super-absorbent polymer, but has a lower basis weight than the other sections of the absorbent body, and it has a belt-shaped elongated shape, with a start point and end point on the absorbent body side edge or near the absorbent body side edge, and with a curved or broken line shape that is convex toward the inside in the widthwise direction. Preferably, as shown in Fig. 1, it has the start point and end point on the side edge of the absorbent body, and has an arc shape that is convex toward the inside of the widthwise direction. However, the shape of the fold-inducing band 8 may be a straight linear shape, or a shape that is partially toward the inside, for example, so long as it is an overall shape from the inside of the absorbent body toward the side edge of the absorbent body which is the outer side. The widthwise direction in this case is the same as the left-right direction. Moreover, the start point and end point of the fold-inducing band 8 is the same as the front edge 8f and the rear edge 8r of the fold-inducing band.

The distance between the right fold-inducing band and the left fold-inducing band in the widthwise direction is preferably narrowest at the center of the crotch region, and widest at the front edges 8f and rear edges 8r of the fold-inducing band.

The crotch region 4C of the absorbent body is the region that contacts with the crotch when the absorbent article is fitted. The front region 4F of the absorbent body is the region of the absorbent body that is more toward the front (abdomen side) than the crotch region 4C. The back region 4R of the absorbent body is the region of the absorbent body that is more toward the back (dorsal side) than the crotch region 4C. The center of the crotch region of the absorbent body is roughly the center in the longitudinal direction of the crotch region 4C. Stated differently, the location where the distance between the right fold-inducing band and the left fold-inducing band in the widthwise direction is narrowest is the center of the crotch region of the absorbent body.

The distance between the right fold-inducing band and the left fold-inducing band in the widthwise direction is not particularly restricted, but at its narrowest it may be, for example, 20 to 90 mm, preferably 25 to 75 mm and more preferably 35 to 60 mm, and at its widest it is preferably approximately matching the width of the absorbent body.

The width of the fold-inducing band is not particularly restricted and may be, for example, 3 to 30 mm, preferably 5 to 25 mm and more preferably 7 to 20 mm. If the width of the fold-inducing band is too narrow, the potential may increase for shifting of the synchronized locations of the fold-inducing bands and the pressured embossing, while conversely if it is too wide, the potential for shifting of the synchronized locations will be lower but there will be more regions where the hydrophilic fibers and the super-absorbent polymer contents are low, possibly reducing the water retention of the absorbent body.

The linear distance between the front edge and the rear edge of the fold-inducing band is not particularly restricted and may be, for example, 50 to 400 mm, preferably 100 to 350 mm and more preferably 150 to 300 mm. If the linear distance between the front edge and the rear edge of the fold-inducing band is too short, the lower crotch region may become tight when the diaper is worn, or conversely if it is too long, the lower crotch region may become loose when the diaper is worn.

The front edge 8f and rear edge 8r of each fold-inducing band are located more toward the outer side in the widthwise direction than the widthwise inside location 9i of the joined area 9 between the side sheet 5 and top sheet 2 at the widthwise inside edge sections of the front edge region and rear edge region of the side sheet. Preferably, as shown in Fig. 1, the front edges 8f and rear edges 8r of the fold-inducing bands are located at the side edges 4r, 4l of the absorbent body, or in other words, the fold-inducing bands reach to the side edges 4r, 4l of the absorbent body.

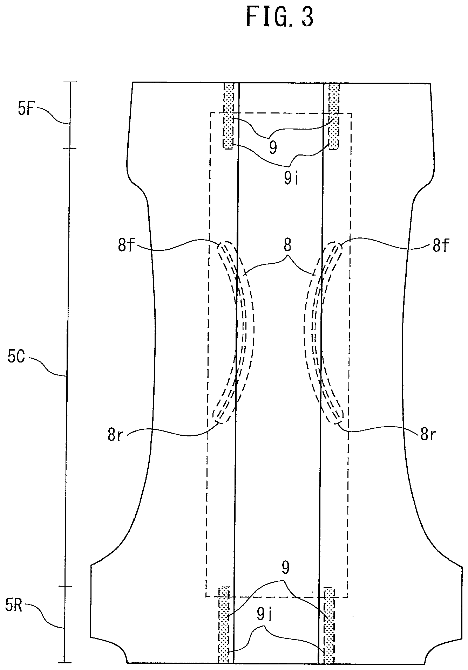

Fig. 3 shows the shapes of the fold-inducing bands according to another embodiment. Fig. 3 omits depiction of some of the members. Each fold-inducing band 8 in Fig. 3 has the front edge 8f and rear edge 8r of the fold-inducing band located more toward the outer side in the widthwise direction than the widthwise inside location 9i of the joined area 9 between the side sheet and top sheet at the widthwise inside edge sections of the front edge region 5F and rear edge region 5R of the side sheet, but they do not reach the side edges 4r, 4l of the absorbent body.

By having the front edge 8f and rear edge 8r of the fold-inducing band located more toward the outer side in the widthwise direction than the widthwise inside location 9i of the joined area 9 between the side sheet 5 and top sheet 2 at the widthwise inside edge sections of the front edge region and rear edge region of the side sheet, it is possible to prevent the absorbent body from folding into the inside in the widthwise direction, near the front edge and rear edge of the leak barriers (that is, the fitting property at the crotch section is excellent) and leakage beyond the leak barriers can be prevented.

The side sheet 5 and top sheet 2 are joined at the widthwise inside edge sections of the front edge region and rear edge region of the side sheet, forming joined areas 9.

The side sheet 5 is separated into a front edge region 5F, a center region 5C and a rear edge region 5R in the longitudinal direction, i.e. the lengthwise direction, the center region 5C being the free edge, i.e. the region where the leak barrier is present, the front edge region 5F being a region where the free edge is not present, which is the region further to the front than the center region, and the rear edge region 5R being a region where the free edge is not present, which is the region further to the rear than the center region. The dimensions of the front edge region 5F in the longitudinal direction may be, for example, 10 to 100 mm, preferably 20 to 85 mm and more preferably 30 to 70 mm. The dimensions of the rear edge region 5R in the longitudinal direction may be, for example, 10 to 100 mm, preferably 20 to 90 mm and more preferably 30 to 80 mm.

The widthwise inside edge section of the front edge region of the side sheet is the region near the widthwise inside edge of the front edge region of the side sheet, and for example, it is the region within 10 mm from the widthwise inside edge. The widthwise inside edge section of the rear edge region of the side sheet is the region near the widthwise inside edge of the rear edge region of the side sheet, and for example, it is the region within 10 mm from the widthwise inside edge.

The joined areas 9 between the side sheet 5 and top sheet 2 are present in the widthwise inside edge sections of the front edge region and rear edge region of the side sheet. The joined areas 9 need only be present in the regions near the widthwise inside edges of the front edge region and the rear edge region of the side sheet, and do not necessarily have to be present up to the widthwise inside edges, and for example, they do not have to be joined within 2 mm and preferably within 1 mm from the widthwise inside edge. The dimensions of each joined area 9 in the longitudinal direction in the front edge region 5F of the side sheet are not particularly restricted and may be, for example, 10 to 100 mm, preferably 20 to 85 mm and more preferably 30 to 70 mm. The dimensions of each joined area 9 in the longitudinal direction in the rear edge region 5R of the side sheet are not particularly restricted and may be, for example, 10 to 100 mm, preferably 20 to 90 mm and more preferably 30 to 80 mm. The dimensions of each joined area 9 in the widthwise direction are not particularly restricted and may be, for example, 1 to 20 mm, preferably 1.5 to 15 mm and more preferably 2 to 10 mm. The dimensions of each joined area 9 in the widthwise direction in the front edge region 5F of the side sheet and the dimensions of each joined area 9 in the widthwise direction in the rear edge region 5R of the side sheet may be the same or different.

The widthwise inside locations 9i of each joined area 9 between the side sheet 5 and the top sheet 2 are preferably at the same distance from the widthwise inside edge of the side sheet in both the front edge region and the rear edge region of the side sheet, but they may instead be different. When the distances of the widthwise inside locations 9i from the widthwise inside edge of the side sheet are different in the front edge region and the rear edge region, the front edge 8f of the fold-inducing band is located more on the outer side in the widthwise direction than the widthwise inside location 9i of the joined area 9 between the side sheet 5 and the top sheet 2 in the widthwise inside edge section of the front edge region 5F of the side sheet, and the rear edge 8r of the fold-inducing band is located more on the outer side in the widthwise direction than the widthwise inside location 9i of the joined area 9 between the side sheet 5 and the top sheet 2 in the widthwise inside edge section of the rear edge region 5R of the side sheet.

The widthwise inside edge of the center region 5Ci of the side sheet is the free edge. An elastic member 7 is attached to the free edge. Fig. 1 shows a mode in which only one elastic member 7 is attached, but a plurality of elastic members may be attached instead. The widthwise inside edge of the center region 5Ci of the side sheet is a free edge, and with the elastic member 7 being attached to the free edge, the center region of the side sheet functions as a leak barrier.

The fold-inducing band 8 includes hydrophilic fibers and a super-absorbent polymer, but the basis weight is lower and the super-absorbent polymer content is lower than the other sections of the absorbent body. By having the basis weight of the fold-inducing band 8 lower than the other sections of the absorbent body, the absorbent body will more easily fold at the fold-inducing band when the absorbent article has been fitted, and as a result it will be possible to prevent folding at inconvenient portions, and to prevent unintended deformation of the absorbent body. A compression groove formed by pressured embossing is provided in the fold-inducing band, but if the super-absorbent polymer is abundantly present at locations that are to undergo pressured embossing, the super-absorbent polymer particles will become highly densified by the pressured embossing, such that the absorbent body may become hardened in the fold-inducing band and the particulate feel of the super-absorbent polymer particles may create a feeling of foreign matter on the wearer side surface and the fitting side surface, increasing the potential burden of the absorbent body on the skin, but if the super-absorbent polymer content is lowered such problems can be eliminated. That is, by providing a compression groove in the fold-inducing band which has a low super-absorbent polymer content, it is possible to maintain softness for the absorbent body.

The basis weight of the absorbent body may be uniform in the regions other than the fold-inducing band, but the basis weight may instead differ in the region more inward in the widthwise direction than the fold-inducing band and the region more outward in the widthwise direction than the fold-inducing band. Preferably, the basis weight of the absorbent body is lower in the region more outward in the widthwise direction than the fold-inducing band, compared to the region more inward in the widthwise direction than the fold-inducing band. The basis weight of the absorbent body in the region more outward in the widthwise direction than the fold-inducing band is preferably 50 to 80 % by weight of the basis weight of the absorbent body in the region more inward in the widthwise direction than the fold-inducing band. If the basis weight of the absorbent body is lower in the region more outward in the widthwise direction than the fold-inducing band compared to the region more inward in the widthwise direction than the fold-inducing band, the lower crotch region will not be loose when the diaper is worn, the outer appearance will be smooth, and the fitting property at the crotch section will be improved.

The region more inward in the widthwise direction than the fold-inducing band is the region defined by the left side edge of the right fold-inducing band, the right side edge of the left fold-inducing band, a straight line connecting the front edge of the right fold-inducing band and the front edge of the left fold-inducing band, and a straight line connecting the rear edge of the right fold-inducing band and the rear edge of the left fold-inducing band.

The regions more outward in the widthwise direction than the fold-inducing bands are the regions defined by the right side edge of the right fold-inducing band, the right side edge of the absorbent body, a straight line extending rightward from a line segment connecting the front edge of the right fold-inducing band and the front edge of the left fold-inducing band, and a straight line extending rightward from a line segment connecting the rear edge of the right fold-inducing band and the rear edge of the left fold-inducing band, and the region defined by the left side edge of the left fold-inducing band, the left side edge of the absorbent body, a straight line extending leftward from a line segment connecting the front edge of the right fold-inducing band and the front edge of the left fold-inducing band, and a straight line extending leftward from a line segment connecting the rear edge of the right fold-inducing band and the rear edge of the left fold-inducing band.

Hereunder, the region more inward in the widthwise direction than the fold-inducing band will be referred to as the "crotch fold inside section", and the region more outward in the widthwise direction than the fold-inducing band will be referred to as the "crotch fold outside section".

Also, the fold-inducing bands will also be referred to as the "low basis weight regions" and the regions other than the fold-inducing bands will be referred to as the "high basis weight regions". When the basis weight of the absorbent body is lower in the region more outward in the widthwise direction than the fold-inducing band compared to the region more inward in the widthwise direction than the fold-inducing band, the fold-inducing band will be referred to as a "low basis weight region", the region more outward in the widthwise direction than the fold-inducing band will also be referred to as a "medium basis weight region", and the region of the absorbent body remaining after removing the region more outward in the widthwise direction than the fold-inducing band and the fold-inducing band will also be referred to as a "high basis weight region".

The basis weight of the absorbent body in the region more inward in the widthwise direction than the fold-inducing band is not restricted but is preferably 100 to 1000 $g/m^2$, more preferably 200 to 900 $g/m^2$ and even more preferably 300 to 800 $g/m^2$. If the basis weight of the absorbent body in the region more inward in the widthwise direction than the fold-inducing band is too low, it may not be possible to maintain the water retention performance as an absorbent body (leading to leakage), and conversely if it is too high, the crotch section may become loose, the outer appearance may not be smooth, and the fitting property at the crotch section may be reduced.

The super-absorbent polymer content in the region more inward in the widthwise direction than the fold-inducing band is not restricted but is preferably 10 to 80 % by weight, more preferably 20 to 70 % by weight and even more preferably 30 to 60 % by weight. If the super-absorbent polymer content in the region more inward in the widthwise direction than the fold-inducing band is too low, it may become impossible to maintain the water retention performance as an absorbent body (leading to leakage), and conversely if it is too high, the absorbent body may harden, a feeling of foreign matter may be produced by the particulate feel of the super-absorbent polymer particles, and the absorbent body may become rigid by swelling during urine absorption.

The basis weight of the absorbent body in the fold-inducing band is preferably 35 to 65 % by weight of the basis weight of the absorbent body in the regions more inward in the widthwise direction than the fold-inducing bands. If the basis weight of the absorbent body in the fold-inducing band is too low, deformation of the absorbent body may take place (due to that section), and when excessive infiltration of body fluids such as urine has occurred, the ability to absorb and retain the body fluids will be reduced and the potential for body fluid leakage will increase. Conversely, if it is too high, the absorbent body will become hardened by the pressured embossing (a feeling of foreign matter will be created by the particulate feel of the super-absorbent polymer particles), increasing the potential to create a burden of the absorbent body on the skin, compared to the other locations.

The basis weight of the hydrophilic fibers in the fold-inducing band is preferably 45 to 80 % by weight of the basis weight of the hydrophilic fibers in the regions more inward in the widthwise direction than the fold-inducing bands. If the basis weight of the hydrophilic fibers in the fold-inducing band is too low, the ability to rapidly absorb body fluids such as urine (initial water-absorption capacity) into the absorbent body may be reduced, increasing the potential for body fluid leakage. Conversely, if it is too high, formation of the fold-inducing band by the pressured embossing may be inadequate (due to the thickness of the hydrophilic fibers), and the ability to control folding into the inside of the absorbent body may be reduced.

The basis weight of the super-absorbent polymer in the fold-inducing bands is preferably 25 to 50 % by weight of the basis weight of the super-absorbent polymer in the regions more inward in the widthwise direction than the fold-inducing bands. If the basis weight of the super-absorbent polymer in the fold-inducing bands is too low, the ability to absorb and retain body fluids such as urine may be reduced, increasing the potential for body fluid leakage. Conversely, if it is too high, the absorbent body will become hardened by the pressured embossing (a feeling of foreign matter will be created by the particulate feel of the super-absorbent polymer particles), increasing the potential to create a burden of the absorbent body on the skin, compared to the other locations.

The super-absorbent polymer content in the fold-inducing band is preferably 10 to 80 % by weight, more preferably 15 to 70 % by weight and even more preferably 20 to 60 % by weight. If the super-absorbent polymer content in the fold-inducing band is too low, the ability to absorb and retain body fluids such as urine may be reduced, increasing the potential for body fluid leakage. Conversely, if it is too high, the absorbent body will become hardened by the pressured embossing (a feeling of foreign matter will be created by the particulate feel of the super-absorbent polymer particles), increasing the potential to create a burden of the absorbent body on the skin, compared to the other locations.

The super-absorbent polymer (hereunder also referred to as "SAP") content is measured in the following manner.

[SAP content measuring method (toluene immersion method)]

<Preparatory materials>

- Absorbent body
- Punch die (punch die equipped with metal blade)
- 250 Mesh nylon net (N-NO.250HD by NBC Industries) (sealed into a 200 × 200 mm bag)
- Toluene
- Aluminum tray
- Forceps
- Filter paper (Advantech, Inc. No.2, 100 mm × 100 mm)
- 3.5 kg/100 cm$^2$ weight
- Electronic scale
- Heat sealer
- Beaker (1000 mL)
- Centrifugal separator (Model HI30 separator by Kokusan Centrifugation Co., Ltd., rotational speed: 850 rpm = 150G)

<Evaluation flow>

(Steps conducted out of draft)

(1) After pulp and SAP layering, tissues coated with a hot-melt adhesive are overlaid to prepare an absorbent body sandwiched by tissues.
(2) Punching is carried out in each sample at each section using a punch die (area: 2.785 cm$^2$) equipped with a metal blade, and approximately 1 g is sampled.

*Sections: Three sections: the crotch fold inside section, crotch fold outside section and fold-inducing band.
The dry weight of the mesh into which each sample is to be placed is measured.

(Steps conducted in draft)

(3) Toluene is placed in the aluminum tray and the punched sample is immersed and rinsed in the toluene, after which it is transferred to a different tray and the upper and lower tissues are peeled off with the forceps.
(4) After lightly draining off the liquid, it is placed in a nylon net and sandwiched with 20 upper and lower filter paper sheets, and subjected to the load of the weight (1 min).
*The dry weight of the nylon net is previously measured (weight A).
(5) After confirming some degree of removal of the toluene, the absorbent body in the nylon net is stirred (broken up) with a dispensing spoon.
(6) Drying is performed in a draft (it may also be dried by hanging with clothespins provided in the draft) (5 hours).
(7) After confirming volatilization of the toluene, the net containing the absorbent body is removed (with appropriate adjustment of the time depending on the state of drying).

(Steps conducted out of draft)

(8) The remaining side of the nylon net is sealed, and the dry weight of the encapsulated absorbent body is measured (weight B).

(8-1) Measurement of water absorption factor

(8-1-1) 1000 mL of ion-exchanged water is added to the beaker.
(8-1-2) The prepared encapsulated bag is immersed so as to touch the bottom of the beaker of (8-1-1).
(8-1-3) The top side of the bag is affixed to the edge of the beaker with a clothespin.
(8-1-4) It is allowed to stand for 1 hour.
(8-1-5) After standing, the bag is lifted and the center of the short side of the bag (5 mm from the top edge and 50 mm from both edges) is sandwiched with clothespins and drained for 15 minutes.
(8-1-6) The weight is measured (weight C).

(8-2) Measurement of water retention factor

(8-2-1) The contents of (8-1-6) are dewatered with a centrifugal separator. Conditions: 850 rpm (150G), time: 90 seconds after reaching constant 850 rpm.
(8-2-2) The weight after dewatering is measured (weight D).

(8-3) The water absorption factor and water retention factor are calculated by the following formulas.

$$\text{Water absorption factor} = (\text{Weight C} - \text{weight A})/(\text{weight B} - \text{weight A})$$

$$\text{Water retention factor} = (\text{Weight D- weight A})/(\text{weight B- weight A})$$

*For both the water absorption factor and water retention factor, the water absorption factor and water retention factor of the nylon net are ignored.

(9) Calibration curve for SAP percentage calculation

The following 3 samples are prepared, and the water absorption factor and water retention factor are calculated in the same manner as (1) to (8) above.

- Pulp: 1 g (SAP percentage: 0%)
- Pulp: 0.5 g, SAP: 0.5 g (SAP percentage: 50%)
- SAP:1 g (SAP percentage: 100%)

(10) A calibration curve is drawn based on the measurement results of (9) above (SAP content based on water retention factor).

*The SAP content can be determined from the water retention factor, by a straight line approximated from 3 points.

(11) The water retention factor for each section of each absorbent body measured in (8) above is inserted in the calibration curve prepared in (10) above, and the SAP content is calculated for each section of each absorbent body.

*Similar to (8), the water absorption factor and water retention factor of the nylon net may be ignored for the water absorption factor and water retention factor calculated in (9), and may therefore be considered to have no effect on the value of the SAP content.

A compression groove 10 is provided in each fold-inducing band 8. A compression groove is a belt-shaped groove formed by compaction. By providing the compression groove 10, the absorbent body will more easily fold at the location where the compression groove has been formed, when the absorbent article has been fitted, and as a result it will be possible to prevent folding at inconvenient portions, and to prevent unintended deformation of the absorbent body. This effect is also exhibited by the low basis weight of the fold-inducing band, but providing the compression grooves can further effectively prevent unintended deformation of the absorbent body. The compression grooves also contribute to preventing deformation of the absorbent body.

The compression grooves have higher density than the other sections of the absorbent body. The density referred to here is the mass per unit volume. The density ($g/cm^3$) is determined by measuring the area ($cm^2$), thickness (cm) and mass (g) of a sample, and dividing the mass (g) by the product of the area ($cm^2$) and the thickness (cm). The density at the other sections of the absorbent body is not restricted but is preferably 0.01 to 0.5 $g/cm^3$, more preferably 0.03 to 0.4 $g/cm^3$ and even more preferably 0.05 to 0.3 $g/cm^3$. If the density of the other sections of the absorbent body is too low, the absorbent body as a whole may become loose, the outer appearance may fail to be smooth, and in particular the fitting property may be reduced at the crotch section. If it is too high, conversely, the absorbent body as a whole may harden, creating a burden on the skin.

The compression groove may be present anywhere in each fold-inducing band so long as it is provided in the fold-inducing band, but preferably the compression groove is provided at roughly the center in the widthwise direction of the fold-inducing band.

The width of the compression groove is not restricted so long as it is smaller than the width of the fold-inducing band, but it is preferably 0.5 to 20 mm, more preferably 1 to 10 mm and even more preferably 1.5 to 5 mm. If the width of the compression groove is too narrow, the bonding strength by pressured embossing may be reduced, the top sheet and the absorbent body that are joined may separate, and actual guiding of folding (at the compacted groove as the origin) may become impossible. If it is too wide, conversely, the fold-inducing band may have a reduced hydrophilic fibers and super-absorbent polymer content, but the rigidity of the pressured embossing can potentially produce a burden on the skin.

The linear distance between the front edge and rear edge of the compression groove is not limited so long as it is the same as or shorter than the linear distance between the front edge and rear edge of the fold-inducing band, but it is preferably 50 to 400 mm, more preferably 100 to 350 mm and even more preferably 150 to 300 mm. If the linear distance between the front edge and rear edge of the compression groove is too short, the lower crotch region may become tight when the diaper is worn. If it is too long, conversely, the lower crotch region may become loose when the diaper is worn.

The compression groove will usually be formed by pressured embossing of the absorbent body with an embossing roll or the like. According to one embodiment, the compression groove is formed by pressured embossing of the absorbent body through the top sheet. In other words, the compression groove may be formed by pressured embossing of the absorbent body and the top sheet together from the top sheet side. As a different embodiment, the absorbent body wrap sheet lies between the absorbent body and the top sheet and between the absorbent body and the back sheet, the compression grooves being formed by pressured embossing of the absorbent body through the top sheet and the absorbent body wrap sheet or through the absorbent body wrap sheet. That is, the compression groove is preferably formed by pressured embossing of the absorbent body and the absorbent body wrap sheet together from the absorbent body wrap sheet side when the surface of the top sheet side of the absorbent body is covered by the absorbent body wrap sheet. The compression groove may also be formed by pressured embossing of the absorbent body, the absorbent body wrap sheet and the top sheet together from the top sheet side.

The top sheet of the absorbent article serves the function of causing liquid excreta from the body, such as urine, to pass through the absorbent body provided on the lower layer, while holding the absorbent body by sandwiching the absorbent body against the back sheet. All or a portion of the top sheet is liquid-permeable, the liquid-permeable regions being formed of a resin film with a plurality of liquid-permeable holes formed therein, or a net-like sheet with a plurality of mesh holes, or a liquid-permeable nonwoven fabric or woven fabric. The aforementioned resin film or net-like sheet may be one formed of polypropylene (PP), polyethylene (PE), polyethylene terephthalate (PET) or the like. Also, a nonwoven fabric used may be a spunlace nonwoven fabric formed from cellulose fibers such as rayon or synthetic resin fibers, or an air-through nonwoven fabric formed of synthetic resin fibers, or the like. Also, the material used may be a biodegradable natural material such as polylactic acid, chitosan, polyalginic acid or the like. In addition, together with formation of the plurality of liquid-permeable holes, a silicone-based or fluorine-based water-repellent lubricant may be applied to render the outer side resistant to adhesion of body fluids.

The back sheet of the absorbent article has the function of preventing outside leakage of fluids such as urine that have been absorbed by the absorbent body, and therefore the material used is one that can prevent outside leakage of such fluids. If the material used prevents passage of fluids but is air permeable, it will be possible to reduce dampness during wear and alleviate unpleasantness during wear. Examples of such materials include liquid-impermeable films composed mainly of polyethylene (PE) or polypropylene (PP), air-permeable films, and composite sheets obtained by layering a liquid-impermeable film onto one side of a spunbond or other type of nonwoven fabric. It is preferred to use a hydrophobic nonwoven fabric, an impermeable plastic film or a laminated sheet comprising a nonwoven fabric and an impermeable plastic film. The material may also be an SMS nonwoven fabric obtained by sandwiching a meltblown nonwoven fabric with high water resistance between high-strength spunbond nonwoven fabrics.

The absorbent body of the absorbent article has the function of absorbing and retaining fluids such as urine, and preferably it has high bulk, is resistant to deformation and has low chemical irritability.

The absorbent body includes hydrophilic fibers and a super-absorbent polymer.

The hydrophilic fibers is not restricted, and may be fluffy pulp, chemical pulp, cellulose fiber, or artificial cellulose fiber such as rayon, acetate or the like.

A superabsorbent polymer (SAP) has a three-dimensional network structure with an appropriately crosslinked water-soluble polymer and therefore absorbs a few ten to a few hundred times its weight in water, but it is essentially water-insoluble and the absorbed water does not emerge even with some degree of pressure application; examples thereof include starch-based, acrylic acid-based and amino acid-based particulate or fibrous polymers.

The absorbent body may be sandwiched or enveloped by an absorbent body wrap sheet. The absorbent body wrap sheet is not restricted and may be a tissue.

The side sheets of the absorbent article form leak barriers.

The material composing each side sheet may be an air-through nonwoven fabric, spunbond fabric, film or porous film, among which an air-through nonwoven fabric is preferred.

The side sheets 5 shown in Fig. 1 and Fig. 2 are provided on both the left and right sides of the absorbent article, their outer contours being essentially the same as the outer sheet 6 at the sections overlapping with the outer sheet 6, and partially overlapping with the side edge sections of the top sheet 2, while the sections not overlapping with the top sheet 2 overlap with the outer sheet 6, and are at least partially joined to it. Joining between the side sheets 5, top sheet 2 and outer sheet 6 may be accomplished, for example, with a hot-melt adhesive or by heat embossing. However, the side sheets 5 are not necessarily limited to the forms shown in Fig. 1 and Fig. 2, and the form may be such that the side edge sections of the side sheets in the widthwise direction wrap around the back side of the absorbent body, together with the top sheet.

The elastic member attached on the free edge of the side sheet is not limited, but may be natural rubber yarn, or synthetic elastic fiber such as polyurethane elastic fiber or polyester elastomer fiber.

The outer sheet composing the absorbent article is provided on the opposite side of the top sheet, outlining the outer shape of the absorbent article. The material composing the outer sheet is not particularly restricted, and may be a nonwoven fabric such as a spunbond-meltblown-spunbond (SMS) nonwoven fabric, point bond nonwoven fabric or spunbonded nonwoven fabric. The outer sheet may be composed of multiple members, and may be formed of, for example, two or more overlaid nonwoven fabrics. In addition, the back sheet may be sandwiched between two or more nonwoven fabrics composing the outer sheet. The outer sheet may include a cover nonwoven fabric (side top sheet) present further on the skin side than the back sheet.

A process for production of the absorbent article of the invention will now be described.

The absorbent body is formed by mixing hydrophilic fibers and a super-absorbent polymer, and layering them. The layering is accomplished in such a manner that the basis weight and SAP content are lower in the region corresponding to the fold-inducing band.

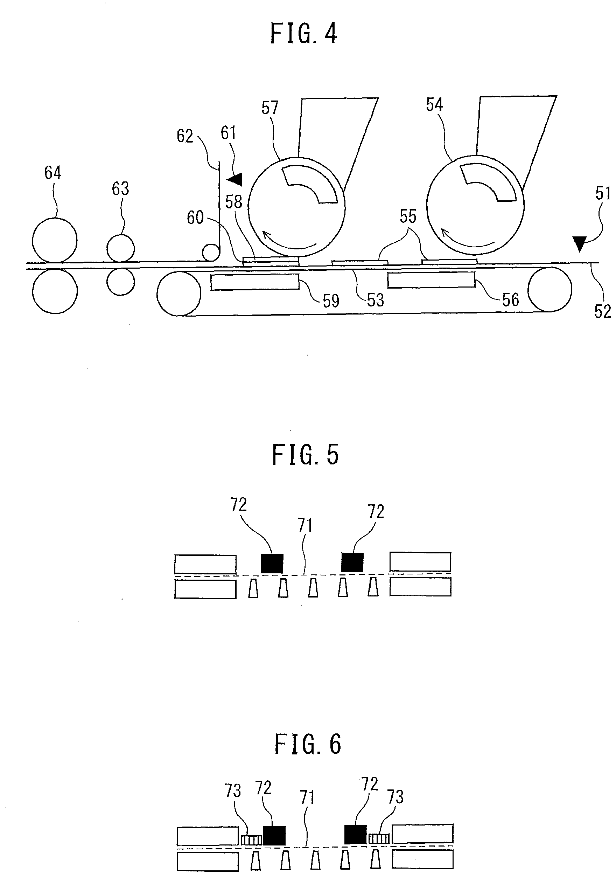

Fig. 4 is a diagram showing an example of a production apparatus for an absorbent body. However, the method for producing the absorbent article of the invention is not limited to the one depicted in Fig. 4.

First, at a coater 51, an adhesive such as a hot-melt adhesive is dispersively coated in a filamentous manner over the entire surface of an absorbent body wrap sheet 52 such as a tissue. The adhesive-coated absorbent body wrap sheet 52 is set on a belt conveyor 53 and conveyed.

At a first layering drum 54, the section corresponding to the high basis weight region (or when a medium basis weight region is to be provided, the section corresponding to the high basis weight region and medium basis weight region, same hereunder) is layered. The layered basis weight is set to be the basis weight of the high basis weight region minus the basis weight of the low basis weight region (i.e. the fold-inducing band). The layering is accomplished by mixing the hydrophilic fibers and super-absorbent polymer, conveying the mixture by air and layering it on an air-permeable mesh screen 71 such as shown in Fig. 5. Non-air permeable protrusions 72 are provided so that the absorbent body is layered in the region corresponding to the fold-inducing band when it is layered. The material for the non-air permeable protrusions 72 is not limited, but for example, a metal plate of aluminum, iron or the like or an elastic solid plate of nylon or rubber may be used. When a medium basis weight region is to be provided, there may also be provided air-permeable control members 73 at the locations corresponding to the medium basis weight regions, as shown in Fig. 6. The locations where the air-permeable control members 73 are provided have lower layered basis weight. The air-permeable control members are not limited, and for example, mesh (for example, metal or elastic solid) plates may be used.

A first layering material 55 layered on the first layering drum 54 is suctioned by a transfer suction box 56 provided under the belt conveyor 53, to transfer it to the adhesive-coated side of the absorbent body wrap sheet 52.

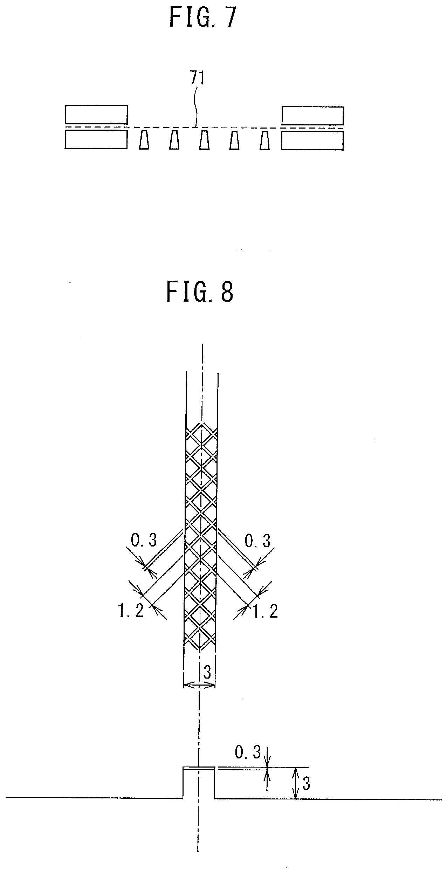

The absorbent body as a whole is then layered at the second layering drum 57. The layered basis weight is set according to the basis weight of the low basis weight region. At the second layering drum 57, layering is performed with a uniform basis weight on the air-permeable mesh screen 71 where the non-air permeable protrusions and air-permeable control members are not present, as shown in Fig. 7.

A second layering material 58 layered on the second layering drum 57 is suctioned by a transfer suction box 59 provided under the belt conveyor 53, to transfer it onto the first layering material 55, to form a layered body 60 of the first layering material 55 and second layering material 58.

Next, at a coater 61, an adhesive such as a hot-melt adhesive is dispersively coated in a filamentous manner over the entire surface of an absorbent body wrap sheet 62 such as a tissue, and the absorbent body wrap sheet 62 is stacked on the layered body 60 so that the coated surface contacts with the layered body 60. The layered body 60 on which the absorbent body wrap sheet 62 has been stacked passes through a pressing device 63, where it is compacted so that the thickness of the high basis weight region becomes constant. The thickness is not limited, but considering that the purpose is as a disposable diaper to be used by a child, it is preferably adjusted to about 3 mm in the high basis weight region.

Next, a pressured embossing roll 64 is used to form compression grooves in the fold-inducing bands. The embossing shape of the pressured embossing roll 64 is not limited and may be a shape such as shown in Fig. 8, for example. The units of the numerical values in Fig. 8 are mm. The embossing strength is not limited, but considering that the purpose is as a disposable diaper to be used by a child, the bonding strength at the joined areas after pressured embossing is preferably adjusted to be 0.065 N/25 mm or greater.

This step produces an absorbent body sandwiched between two absorbent body wrap sheets.

Next, the outer sheet, back sheet, absorbent body, top sheet and pair of side sheets are overlaid by a common method to produce an absorbent article.

An example of a method for producing an absorbent article according to the invention was explained above, but the method for producing an absorbent article of the invention is not limited to this method.

In a disposable diaper of the prior art having a center absorbent body and slits on side absorbent bodies, the presence of the slits lowers the strength (form retention) of the absorbent bodies themselves at those locations, while the slits themselves do not have the body fluid absorption properties of an absorbent body. Consequently, after the absorbent article has been fitted, the absorbent body deforms around the slits as centers, after activity such as movement of the legs, tending to cause shifting of the diaper (freeing by splitting of the absorbent body), which lowers the ability to absorb and retain body fluids such as urine centered around the peripheral sections, increasing the possibility of body fluid leakage, and resulting in reduced leakproofness of the absorbent article. Moreover, despite the presence of the slits, since the absorbent body does not have a specific folded structure at the front body region of the absorbent body, the absorbent body folds in at the non-slit locations, often making it impossible to ensure the desired absorption area.

In contrast, the absorbent article of the invention provides fold guiding regions in the absorbent body and provides compression grooves in the fold guiding regions, whereby the absorbent body becomes resistant to deformation, the outer appearance is smooth when worn, and the fitting property at the lower crotch region is excellent.

Examples

The absorbent body production apparatus shown in Fig. 4 was used to produce an absorbent body. The obtained absorbent body was used to produce a disposable diaper by a common method, and it was evaluated.
The following starting materials were used in the examples and comparative examples.

Hydrophilic fiber: Wood fluff pulp (NBKP)
Super-absorbent polymer: SAP AQUA KEEP SA60S by Sumitomo Seika Chemicals Co., Ltd.
Hot-melt adhesive: Styrene-based thermoplastic elastomer hot-melt adhesive by Henkel Japan, Ltd.
Tissue: Unicharm Kokko Nonwoven Co., Ltd. (16 g/m$^2$)

Example 1

At the first layering drum, a conveyed tissue was coated with a hot-melt adhesive at 5 g/m$^2$, and previously weighed 90 g/m$^2$ of pulp and 140 g/m$^2$ of SAP were layered over it. At the locations corresponding to the fold-inducing band, non-air permeable protrusions 72 were provided as shown in Fig. 5, so that they were not layered.
At the second layering drum, previously weighed 160 g/m$^2$ of pulp and 80 g/m$^2$ of SAP were layered, and a tissue coated with a hot-melt adhesive at 5 g/m$^2$ was layered thereover to form an absorbent body sandwiched between tissues.
After compressing the thickness at the high basis weight sections to 3 mm with a pressing device, an embossing roll with the embossing shape shown in Fig. 8 was used for pressured embossing to form compression grooves at locations corresponding to the fold-inducing bands. The bonding strength at the joined areas after pressured embossing was adjusted to 0.065 N/25 mm.
The fold-inducing bands and the compression grooves extended forward and backward from the center of the crotch region, as shown in Fig. 1, and the distance between the right fold-inducing band and the left fold-inducing band in the widthwise direction was narrow at the center of the crotch region and wider as it receded from the center of the crotch region, so that it elongated up to the side edges of the absorbent body. This procedure yielded an absorbent body having the crotch fold inside sections and crotch fold outside sections as high basis weight regions (pulp: 250 g/m$^2$, SAP: 220 g/m$^2$) and the fold-inducing bands as low basis weight regions (pulp: 160 g/m$^2$, SAP: 80 g/m$^2$). The low basis weight regions of the fold-inducing bands each comprise a high density region subjected to pressured embossing at the center section, and low density regions without embossing around the periphery. The outer dimensions of the absorbent body were 420 mm length and 120 mm width, the width of each fold-inducing band was 10 mm, the distance between the two fold-inducing bands in the widthwise direction was 35 mm at the narrowest and the linear distance between the front edges and rear edges of the fold-inducing bands was 220 mm.
The prepared absorbent body sandwiched by tissues was stacked on an outer sheet and a back sheet, a top sheet was stacked over the absorbent body sandwiched by tissues, and a pair of side sheets were stacked over it, to fabricate a disposable diaper such as shown in Fig. 1.

Example 2

A disposable diaper was fabricated in the same manner as Example 1, except that at the first layering drum, the positions of the non-air permeable protrusions of the first layering drum are adjusted for the layering so that, as shown in Fig. 3, the front edges and back edges of the fold-inducing bands were located more toward the inside in the widthwise direction than the side edges of the absorbent body, and located more toward the outer sides in the widthwise direction than the widthwise inside locations of the joined areas between the side sheets and top sheet at the widthwise inside edge sections of the front edge regions and rear edge regions of the side sheets. The distance of the front edges and back edges of the fold-inducing bands from the side edges of the absorbent body was 10 mm, and the linear distance of the front edges and rear edges of the fold-inducing bands was 195 mm.

Example 3

A disposable diaper was fabricated in the same manner as Example 1, except that at the first layering drum, the air-permeable control members opened with meshes were situated at locations corresponding to the crotch fold outside sections (see Fig. 6), and adjustment was for layering with 25 g/m$^2$ of pulp and 40 g/m$^2$ of SAP.

Example 4

Layering was with 130 g/m$^2$ of pulp and 160 g/m$^2$ of SAP at the first layering drum. However, the non-air permeable protrusions were provided at locations corresponding to the fold-inducing bands, the air-permeable control members opened with meshes were situated at locations corresponding to the crotch fold outside sections to avoid layering there (see Fig. 6), and adjustment was for layering with 25 g/m$^2$ of pulp and 30 g/m$^2$ of SAP. Layering was with 120 g/m$^2$ of pulp and 60 g/m$^2$ of SAP at the second layering drum. The procedure was otherwise conducted in the same manner as Example 1 to fabricate a disposable diaper.

Example 5

Layering was with 50 g/m$^2$ of pulp and 120 g/m$^2$ of SAP at the first layering drum. However, the non-air permeable protrusions were provided at locations corresponding to the fold-inducing bands, the air-permeable control members opened with meshes were situated at locations corresponding to the crotch fold outside sections to avoid layering there (see Fig. 6), and adjustment was for layering with 25 g/m$^2$ of pulp and 50 g/m$^2$ of SAP. Layering was with 200 g/m$^2$ of pulp and 100 g/m$^2$ of SAP at the second layering drum. The procedure was otherwise conducted in the same manner as Example 1 to fabricate a disposable diaper.

Comparative Example 1

A disposable diaper was fabricated in the same manner as Example 1, except that at the first layering drum, the positions of the non-air permeable protrusions of the first layering drum were adjusted for the layering so that the front edges and back edges of the fold-inducing bands were located even further toward the inside in the widthwise direction than the widthwise inside locations of the joined areas between the side sheets and top sheet at the widthwise inside edge sections of the front edge regions and rear edge regions of the side sheets.

Comparative Example 2

Layering was with 250 g/m$^2$ of pulp and 220 g/m$^2$ of SAP at the first layering drum. At the locations corresponding to the fold-inducing bands, non-air permeable protrusions were provided as shown in Fig. 5, so that they were not layered. The second layering drum step was omitted. The procedure was otherwise conducted in the same manner as Example 1 to fabricate a disposable diaper. The disposable diaper of Comparative Example 2 had the crotch fold inside section and crotch fold outside sections as the high basis weight regions (pulp: 250 g/m$^2$, SAP: 220 g/m$^2$), and slits without basis weight were present at locations corresponding to the fold-inducing bands.

Comparative Example 3

Layering was with 250 g/m$^2$ of pulp and 220 g/m$^2$ of SAP at the first layering drum. However, at the layering sections of the first layering drum, an air-permeable mesh screen without non-air permeable protrusions or air-permeable control members was used, as shown in Fig. 7. The second layering drum step was omitted. The procedure was otherwise conducted in the same manner as Example 1 to fabricate a disposable diaper. The disposable diaper of Comparative Example 3 had all of the regions at locations corresponding to the crotch fold inside section, crotch fold outside sections and fold-inducing bands as the high basis weight regions (pulp: 250 g/m$^2$, SAP: 220 g/m$^2$).

Comparative Example 4

A disposable diaper was fabricated in the same manner as Example 1, except that no pressured embossing was carried out. The disposable diaper of Comparative Example 4 lacked compression grooves.

Comparative Example 5

A disposable diaper was fabricated in the same manner as Comparative Example 3, except that no pressured embossing was carried out. The disposable diaper of Comparative Example 5 lacked fold-inducing bands and compression grooves.

Comparative Example 6

A disposable diaper was fabricated in the same manner as Comparative Example 2, except that for the mesh screen shape at the layering section of the first layering drum there was used a mesh screen with a shape with the crotch fold outside sections eliminated (an "hourglass" shape). The disposable diaper of Comparative Example 6 lacked both the crotch fold outside sections and the fold-inducing bands in the absorbent body.

The disposable diapers fabricated in the examples and comparative examples were evaluated in terms of folding into the inside of the absorbent body, softness (load on the skin) and deformation (positional shifting of the diaper). The evaluated properties and evaluation methods were as follows. The evaluation results are shown in Table 1.

[Folding into inside of absorbent body] (Confirmation by actual fitted state on babies]

The disposable diapers to be evaluated were fitted onto 6 babies, and the outer appearance of each disposable diaper after 20 minutes of free activity, and folding in from the top sheet (skin side) of the diaper to the inside of the absorbent body after removal, were evaluated as follows.
Number of individuals with folding in: 0 to 1 (G), 2 to 3 (F), ≥4 (P)

[Softness (load on skin)] (Confirmation by opinion of wearer (parent))

The disposable diapers to be evaluated were fitted onto 6 babies, the feel on the top sheet (skin side) of the diaper was confirmed by the wearers (parents), and the opinion on hardness by the pressured embossing was evaluated as follows.
Number of individuals feeling "hardness": 0 to 1 (G), 2 to 3 (F), ≥4 (P)

[Deformation (positional shifting of diaper)] (Confirmation by actual fitted state on babies)

The disposable diapers to be evaluated were fitted onto 6 babies, and after 10 minutes of free activity, 100 cc of 0.9% saline solution was injected into the crotch section, this was followed by another 10 minutes of free activity, and the state of slipping or lifting of the absorbent body from the top sheet (skin) side of the diaper after removal of the disposable diaper was confirmed, with a judgment of "deformation" if the degree of shifting of the absorbent body at the crotch section was large (shifting of 10 mm or more in the widthwise direction compared to the position before use) or if the top sheet was in a lifted state (a difference of 20 mm or more from the position before use), and evaluated as follows.
Number of individuals with deformation: 0 to 1 (G), 2 to 3 (F), ≥4 (P)

[Table 1]

Table 1

| | Crotch fold inside basis weight | | SAP content (wt%) | Crotch fold outside basis weight | | SAP content (wt%) | Fold-inducing band basis weight | | SAP content (wt%) | Compression groove | Fold-inducing band shape[a] | Folding into inside of absorbent body | Softness (Burden on skin) | Deformation (Positional shifting of diaper) |
|---|---|---|---|---|---|---|---|---|---|---|---|---|---|---|
| | Pulp (g/m²) | SAP (g/m²) | | Pulp (g/m²) | (g/m²) | | Pulp (g/m²) | SAP (g/m²) | | | | | | |
| Example 1 | 250 | 220 | 47% | 250 | 220 | 47% | 160 | 80 | 33% | Present | 1 | G | G | G |
| Example 2 | 250 | 220 | 47% | 250 | 220 | 47% | 160 | 80 | 33% | Present | 2 | G | G | G |
| Example 3 | 250 | 220 | 47% | 185 | 120 | 39% | 160 | 80 | 33% | Present | 1 | G | G | G |
| Example 4 | 250 | 220 | 47% | 145 | 90 | 38% | 120 | 60 | 33% | Present | 1 | G | G | G |
| Example 5 | 250 | 220 | 47% | 225 | 150 | 40% | 200 | 100 | 33% | Present | 1 | G | G | G |
| Comp. Ex. 1 | 250 | 220 | 47% | 250 | 220 | 47% | 160 | 80 | 33% | Present | 3 | P | G | G |
| Comp. Ex. 2 | 250 | 220 | 47% | 250 | 220 | 47% | 0 | 0 | - | Absent | 1 | G | F | P |
| Comp. Ex. 3 | 250 | 220 | 47% | 250 | 220 | 47% | 250 | 220 | 47% | Present | 1 | G | P | G |
| Comp. Ex. 4 | 250 | 220 | 47% | 250 | 220 | 47% | 160 | 80 | 33% | Absent | 4 | P | G | P |
| Comp. Ex. 5 | 250 | 220 | 47% | 250 | 220 | 47% | 250 | 220 | 47% | Absent | 4 | P | G | P |
| Comp. Ex. 6 | 250 | 220 | 47% | 0 | 0 | - | 0 | 0 | - | Absent | 4 | P | G | G |

a) Numbers for fold-inducing band shapes
1: Front edge and rear edge of fold-inducing band present at side edges of absorbent body
2: Front edge and rear edge of fold-inducing band present more toward the outside in the widthwise direction than the widthwise inside locations of the joined areas between side sheets and top sheet
3: Front edge and rear edge of fold-inducing band present more toward the inside in the widthwise direction than the widthwise inside locations of the joined areas between side sheets and top sheet
4: Fold-inducing band itself not present

Example 1 is an example in which the crotch fold inside sections and crotch fold outside sections were set as high basis weight regions, the basis weight of the fold-inducing bands were reduced, and the compression grooves by pressured embossing were formed up to the side edges of the absorbent body. By reducing the basis weight of the fold-inducing bands and carrying out pressured embossing, it is possible to maintain the absorption performance of the absorbent body near the fold-inducing bands, and prevent deformation of the absorbent body. In addition, by reducing the SAP content of the fold-inducing bands in addition to reducing the basis weight, it is possible to increase the density of SAP particles by pressured embossing, to avoid hardening of the absorbent body in the fold-inducing bands, maintaining the same flexibility as the other locations, and to reduce the burden on the skin. Furthermore, if the fold-inducing bands extend radially from the crotch folding sections toward the lengthwise direction and reach to the side edges of the absorbent body, folding into the inside of the absorbent body at the front edges and rear edges of the leak barriers may be controlled, and leakage beyond the leak barriers can be prevented.

Example 2 is an example having the construction of Example 1 wherein the fold-inducing bands formed by pressured embossing are more toward the inside in the widthwise direction than the side edges of the absorbent body, and more toward the outer sides in the widthwise direction than the widthwise inside locations of the joined areas between the side sheets and top sheet at the widthwise inside edge sections of the front edge regions and rear edge regions of the side sheets. By being more toward the outer sides in the widthwise direction than the widthwise inside locations of the joined areas between the side sheets and the top sheet at the widthwise inside edge sections of the front edge regions and rear edge regions of the side sheet, an effect is exhibited that is essentially the same as Example 1, minimizing folding into the inside of the absorbent body at the front edges and rear edges of the leak barriers, and allowing prevention of leakage beyond the leak barriers.

Example 3 is an example with the construction of Example 1 wherein the basis weight is reduced at the crotch fold outside sections. Although the basis weight is slightly reduced at the crotch fold outside sections, the effect exhibited is essentially the same as in Example 1. This example may be modified according to the purpose and use of the absorbent article.

Example 4 is an example with the construction of Example 1 wherein the basis weights of the crotch fold outside sections and the fold-inducing bands are further reduced compared to Example 3. By reducing the basis weights of the crotch fold outside section and the fold-inducing bands, it is possible to slightly reduce the absorption volume of the absorbent body as a whole compared to Example 1, but by pressured embossing of the fold-inducing bands it is possible to prevent deformation of the absorbent body. In addition, by reducing the SAP content of the fold-inducing bands in addition to reducing the basis weight, it is possible to increase the density of SAP particles by pressured embossing, to avoid hardening of the absorbent body in the fold-inducing bands, maintaining the same flexibility as the other locations, and to reduce the burden on the skin. This example may be modified according to the purpose and use of the absorbent article.

Example 5 is an example with the construction of Example 1 wherein the basis weight of the crotch fold outside sections is lower than in Example 1 but higher than in Example 3, and the basis weight of the fold-inducing bands is higher than Example 1 or Example 3. By increasing the basis weights of the crotch fold outside sections and the fold-inducing bands compared to Example 3, it is possible to increase the absorption volume of the absorbent body as a whole compared to Example 3, but by pressured embossing of the fold-inducing bands it is possible to prevent deformation of the absorbent body. By increasing the basis weight of the fold-inducing bands, the total amount of SAP occupying those locations also increases and pressured embossing causes the absorbent body to be slightly hardened in the fold-inducing bands of the SAP particles, but approximately the same flexibility as the other sections is maintained, and the burden on the skin can be alleviated. This example may be modified according to the purpose and use of the absorbent article.

Comparative Example 1 is an example having the construction of Example 1 wherein the front edges and back edges of the fold-inducing bands are more toward the inside in the widthwise direction than the widthwise inside locations of the joined areas between the side sheets and top sheet at the widthwise inside edge sections of the front edge regions and rear edge regions of the side sheets. If the form is such that the front edges and back edges of the fold-inducing bands are even more toward the inside in the widthwise direction than the widthwise inside locations of the joined areas between the side sheets and the top sheet at the widthwise inside edge sections of the front edge regions and rear edge regions of the side sheet, this may result in folding into the inside of the absorbent body at the front edges and rear edges of the leak barriers, and urine may not drop down into the conventional absorbent body region but instead may drop onto the leak barriers, often leading to leakage beyond the leak barriers.

Comparative Example 2 is an example in which the basis weight of the fold-inducing bands of Example 1 was eliminated and the fold-inducing bands were used as the slits. If the fold-inducing bands reach to the side edges of the absorbent body, it is possible to control folding into the inside of the absorbent body, but deformation may occur near the site of urination near the crotch fold section due to the slits where the absorbent body (pulp/SAP) is not present, and similar to the prior art example, since the slits themselves do not exhibit body fluid absorption performance as an absorbent body, the ability to absorb and retain body fluids such as urine will be reduced, the potential for body fluid leakage will increase, and as a result the leakproofness (absorption volume) of the absorbent article will be lowered.

Comparative Example 3 is an example in which the basis weight of the fold-inducing bands of Example 1 is a high basis weight, similar to the basis weight of the crotch fold inside section and the basis weight of the crotch fold outside sections. If the fold-inducing bands have a high basis weight, then the increased density of the SAP particles due to the pressured embossing will harden the absorbent body in the fold-inducing bands (producing a feeling of foreign matter by the particulate feel of the SAP), more likely resulting in a greater burden on the skin by the absorbent body, compared to the other locations.

Comparative Example 4 is an example with the construction of Example 1, wherein pressured embossing of the fold-inducing bands is not performed. If there are no compression grooves created by pressured embossing, it will be difficult to control folding into the inside of the absorbent body along the fold-inducing bands, more likely leading to leakage beyond the leak barriers, while deformation of the absorbent body may occur centered around the area near the fold-inducing bands of the low basis weight regions, tending to result in positional shifting of the diaper (freeing by splitting of the absorbent body), which lowers the ability to absorb and retain body fluids such as urine centered around the peripheral sections, increasing the possibility of body fluid leakage, and resulting in reduced leakproofness of the absorbent article.

Comparative Example 5 is an example in which all of the regions of the absorbent body are high basis weight regions, similar to Comparative Example 3, but unlike Comparative Example 3 it has no compression grooves by pressured embossing, and therefore although hardening of the absorbent body does not occur, it is not possible to control folding into the inside of the absorbent body along the fold-inducing bands, increasing the likelihood of leakage beyond the leak barriers, while also tending to result in positional shifting of the diaper (freeing by splitting of the absorbent body), which lowers the ability to absorb and retain body fluids such as urine, centered around the peripheral sections, increasing the possibility of body fluid leakage, and resulting in reduced leakproofness of the absorbent article.

Comparative Example 6 has an "hourglass" form with the crotch fold outside section of the absorbent body eliminated. With a hourglass shape, the regions corresponding to the fold-inducing bands are absent, and deformation of the absorbent body centered around those locations does not occur, but since pressured embossing is absent it becomes impossible to control folding into the inside of the absorbent body along the fold-inducing bands, increasing the likelihood of leakage beyond the leak barriers, while the hourglass shape of the absorbent body lowers the overall absorption volume, thereby lowering the ability to absorb and retain body fluids such as urine, increasing the possibility of body fluid leakage, and resulting in reduced leakproofness of the absorbent article.

Industrial Applicability

The absorbent article of the invention can be suitably used as a disposable diaper for infants, a disposable diaper for children, training pants, a disposable diaper for adults, an incontinence pad, or the like.

Explanation of Symbols

1 Absorbent article
2 Top sheet
3 Back sheet
4 Absorbent body
5 Side sheet
6 Outer sheet
7 Elastic member
8 Fold-inducing band
9 Joined area
10 Compression groove
51 Coater
52 Absorbent body wrap sheet
53 Belt conveyor
54 First layering drum
55 First layering material
56 Transfer suction box
57 Second layering drum
58 Second layering material
59 Transfer suction box

60 Layered body
61 Coater
62 Absorbent body wrap sheet
63 Pressing device
64 Pressured embossing roll
71 Air permeable mesh screen
72 Non-air permeable protrusion
73 Air-permeable control member

**Claims**

1. Diaper comprising a liquid-permeable top sheet (2), a liquid-impermeable back sheet (3), an absorbent body (4) lying between them, a pair of left and right side sheets (5) forming leak barriers, and an outer sheet (6),

   the absorbent body (4) including hydrophilic fibers and a super-absorbent polymer,
   each side sheet (5) consisting of a front edge region (5F), a center region (5C) and a rear edge region (5R), a widthwise outside region (50) of the side sheet (5) being joined with at least one of the top sheet (2), back sheet (3) and outer sheet (6), the side sheet (5) being joined with the top sheet (2) at widthwise inside edge sections of the front edge region (5F) and the rear edge region (5R) of the side sheet (5), a widthwise inside edge of the center region of the side sheet (5) being a free edge, wherein an elastic member (7) is attached at the free edge and the center region of the side sheet (5) functions as a leak barrier, and
   the absorbent body (4) consisting of a front region (4F), a crotch region (4C) and a back region (4R), there being provided in the absorbent body a pair of left and right fold-inducing bands (8, 8) extending both forward and backward from a center of the crotch region (4C), a distance between the right fold-inducing band (8) and the left fold-inducing band (8) in the widthwise direction being wider at front edges (8f) and rear edges (8r) of the fold-inducing bands (8, 8) compared to the center of the crotch region (4C), the front edges (8f) and rear edges (8r) of the fold-inducing bands (8, 8) being located more toward an outer side in a widthwise direction than widthwise inside locations of the joined areas between the side sheets (5) and the top sheet (2) at the widthwise inside edge sections of the front edge regions (5F) and rear edge regions (5R) of the side sheets (5), and the fold-inducing bands (8, 8) including hydrophilic fibers and a super-absorbent polymer, but having a lower basis weight and a lower super-absorbent polymer content than the other sections of the absorbent body (4), wherein a compression groove (10) is formed in each fold-inducing band (8, 8), the compression groove (10) having higher density than the other sections of the absorbent body (4); wherein the front edges (8f) and rear edges (8r) of the fold-inducing bands (8, 8) are located on the side edges of the absorbent body (4), wherein the fold-inducing bands (8, 8) have arc shapes that are convex toward the widthwise center of the absorbent body (4).

2. Diaper according to claim 1, wherein the basis weight of the absorbent body (4) is lower in the regions more outward in the widthwise direction than the fold-inducing bands (8, 8), compared to the regions more inward in the widthwise direction than the fold-inducing bands (8, 8).

3. Diaper according to any one of claims 1 to 2, wherein the basis weight of the absorbent body (4) in the fold-inducing bands (8, 8) is 35 to 65 % by weight of the basis weight of the absorbent body (4) in the regions more inward in the widthwise direction than the fold-inducing bands (8, 8).

4. Diaper according to any one of claims 1 to 3, wherein the basis weight of the hydrophilic fibers in the fold-inducing bands (8, 8) is 45 to 80 % by weight of the basis weight of the hydrophilic fibers in the regions more inward in the widthwise direction than the fold-inducing bands (8, 8), and the basis weight of the super-absorbent polymer in the fold-inducing bands (8, 8) is 25 to 50 % by weight of the basis weight of the super-absorbent polymer in the regions more inward in the widthwise direction than the fold-inducing bands (8, 8).

5. Diaper according to any one of claims 1 to 4, wherein the basis weight of the absorbent body (4) in the regions more outward in the widthwise direction than the fold-inducing bands (8, 8) is 50 to 80 % by weight of the basis weight of the absorbent body (4) in the regions more inward in the widthwise direction than the fold-inducing bands (8, 8).

**Patentansprüche**

1. Windel umfassend eine flüssigkeitsdurchlässige Oberlage (2), eine flüssigkeitsundurchlässige Unterlage (3), einen absorbierenden Körper (4), der zwischen ihnen liegt, ein Paar von linken und rechten Seitenlagen (5), die Auslaufsperren ausbilden, und eine äußere Lage (6),

   der absorbierende Körper (4) umfassend wasserbindende Fasern und ein super-absorbierendes Polymer, jede Seitenlage (5) bestehend aus einem vorderen Kantenbereich (5F), einem Mittelbereich (5C) und einem hinteren Kantenbereich (5R), einem breitseitigen Außenbereich (50) der Seitenlage (5), der mit wenigstens einer der Oberlage (2), Unterlage (3) und Außenlage (6) verbunden ist, der Seitenlage (5), die mit der Oberlage (2) an breitseitigen Innenkantenabschnitten des vorderen Kantenbereichs (5F) und des hinteren Kantenbereichs (5R) der Seitenlage (5) verbunden ist, einer breitseitigen Innenkante des Mittelbereichs der Seitenlage (5), die eine freie Kante ist, wobei ein elastisches Element (7) an der freien Kante befestigt ist und der Mittelbereich der Seitenlage (5) als eine Auslaufsperre fungiert, und

   der absorbierende Körper (4) bestehend aus einem vorderen Bereich (4F), einem Schrittbereich (4C) und einem hinteren Bereich (4R), der dort in dem absorbierenden Körper über ein Paar von linken und rechten faltungseinleitenden Bändern (8, 8) verfügt, die sich sowohl nach vorne als auch nach hinten von einer Mitte des Schrittbereichs (4C) erstrecken, einem Abstand zwischen dem rechten faltungs-einleitenden Band (8) und dem linken faltungs-einleitenden Band (8) in der Breitenrichtung, der an vorderen Kanten (8f) und hinteren Kanten (8r) der faltungs-einleitenden Bändern (8, 8) größer ist verglichen mit der Mitte des Schrittbereichs (4C) ist, den vorderen Kanten (8f) und hinteren Kanten (8r) der faltungs-einleitenden Bänder (8, 8), die näher an einer Außenseite in einer Breitenrichtung angeordnet sind als breitseitige Innenpositionen der verbundenen Flächen zwischen den Seitenlagen (5) und der Oberlage (2) an den breitseitigen Innenkantenabschnitten der vorderen Kantenbereiche (5F) und hinteren Kantenbereiche (5R) der Seitenlagen (5), und den faltungs-einleitenden Bändern (8, 8), die wasserbindende Fasern und ein super-absorbierendes Polymer umfassen, aber mit einem geringeren Grundgewicht und einem niedrigeren super-absorbierenden Polymer Gehalt als die anderen Abschnitte des absorbierenden Körpers (4), wobei eine Kompressionsnut (10) in jedem faltungs-einleitenden Band (8, 8) ausgebildet ist, die Kompressionsnut (10) mit höherer Dichte als die anderen Abschnitte des absorbierenden Körpers (4); wobei die vorderen Kanten (8f) und hinteren Kanten (8r) der faltungs-einleitenden Bänder (8, 8) an den Seitenkanten des absorbierenden Körpers (4) angeordnet sind, wobei die faltungs-einleitenden Bänder (8, 8) Bogenformen aufweisen, die konvex in Richtung der breitseitigen Mitte des absorbierenden Körpers (4) sind.

2. Windel gemäß Anspruch 1, wobei das Grundgewicht des absorbierenden Körpers (4) in den Bereichen weiter außerhalb in der Breitenrichtung als die faltungs-einleitenden Bänder (8, 8) geringer ist verglichen mit den Bereichen weiter innerhalb in der Breitenrichtung als die faltungs-einleitenden Bänder (8, 8).

3. Windel gemäß einem der Ansprüche 1 bis 2, wobei das Grundgewicht des absorbierenden Körpers (4) in den faltungs-einleitenden Bändern (8, 8) 35 bis 65 Gewichtsprozent des Grundgewichts des absorbierenden Körpers (4) in den Bereichen weiter innerhalb in der Breitenrichtung als die faltungs-einleitenden Bänder (8, 8) beträgt.

4. Windel gemäß einem der Ansprüche 1 bis 3, wobei das Grundgewicht der wasserbindenden Fasern in den faltungs-einleitenden Bändern (8, 8) 45 bis 80 Gewichtsprozent des Grundgewichts der wasserbindenden Fasern in den Bereichen weiter innerhalb in der Breitenrichtung als die faltungs-einleitenden Bänder (8, 8) beträgt, und das Grundgewicht des super-absorbierenden Polymers in den faltungs-einleitenden Bändern (8, 8) 25 bis 50 Gewichtsprozent des Grundgewichts des super-absorbierenden Polymers in den Bereichen weiter innerhalb in der Breitenrichtung als die faltungs-einleitenden Bänder (8, 8) beträgt.

5. Windel gemäß einem der Ansprüche 1 bis 4, wobei das Grundgewicht des absorbierenden Körpers (4) in den Bereichen weiter außerhalb in der Breitenrichtung als die faltungs-einleitenden Bänder (8, 8) 50 bis 80 Gewichtsprozent des Grundgewichts des absorbierenden Körpers (4) in den Bereichen weiter innerhalb in der Breitenrichtung als die faltungs-einleitenden Bänder (8, 8) beträgt.


**Revendications**

1. Couche comprenant une feuille supérieure perméable au liquide (2), une feuille arrière imperméable au liquide (3), un corps absorbant (4) situé entre elles, une paire de feuilles latérales gauche et droite (5) formant des barrières antifuites, et une feuille extérieure (6),

le corps absorbant (4) comprenant des fibres hydrophiles et un polymère superabsorbant,

chaque feuille latérale (5) consistant en une région de bord avant (5F), une région centrale (5C) et une région de bord arrière (5R), une région extérieure dans le sens de la largeur (50) de la feuille latérale (5) étant reliée à au moins une parmi la feuille supérieure (2), la feuille arrière (3) et la feuille extérieure (6), la feuille latérale (5) étant reliée à la feuille supérieure (2) au niveau de sections de bord intérieures dans le sens de la largeur de la région de bord avant (5F) et la région de bord arrière (5R) de la feuille latérale (5), un bord intérieur dans le sens de la largeur de la région centrale de la feuille latérale (5) étant un bord libre, dans laquelle un élément élastique (7) est attaché au niveau du bord libre et la région centrale de la feuille latérale (5) fonctionne comme une barrière antifuites, et

le corps absorbant (4) consistant en une région avant (4F), une région d'entrejambe (4C) et une région arrière (4R), il étant prévu dans le corps absorbant une paire de bandes induisant des plis à gauche et à droite (8, 8) s'étendant vers l'avant et vers l'arrière à partir d'un centre de la région d'entrejambe (4C), une distance entre la bande induisant des plis à droite (8) et la bande induisant des plis à gauche (8) dans la direction de la largeur étant plus grande au niveau des bords avant (8f) et bords arrière (8r) des bandes induisant des plis (8, 8) comparée au centre de la région d'entrejambe (4C), les bords avant (8f) et bords arrière (8r) des bandes induisant des plis (8, 8) étant situés plus vers un côté extérieur dans une direction de la largeur que des endroits intérieurs dans le sens de la largeur des zones reliées entre les feuilles latérales (5) et la feuille supérieure (2) au niveau des sections de bord intérieures dans le sens de la largeur des régions de bord avant (5F) et des régions de bord arrière (5R) des feuilles latérales (5), et les bandes induisant des plis (8, 8) comportant des fibres hydrophiles et un polymère superabsorbant, mais ayant un poids de base inférieur et une teneur en polymère superabsorbant inférieure à ceux des autres sections du corps absorbant (4),

dans laquelle une rainure de compression (10) est formée dans chaque bande induisant des plis (8, 8), la rainure de compression (10) ayant une densité supérieure à celle des autres sections du corps absorbant (4) ; dans laquelle les bords avant (8f) et les bords arrière (8r) des bandes induisant des plis (8, 8) sont situés sur les bords latéraux du corps absorbant (4), dans laquelle les bandes induisant des plis (8, 8) ont des formes en arc qui sont convexes vers le centre dans le sens de la largeur du corps absorbant (4).

2. Couche selon la revendication 1, dans laquelle le poids de base du corps absorbant (4) est inférieur dans les régions plus vers l'extérieur dans la direction de la largeur que les bandes induisant des plis (8, 8), comparé aux régions plus vers l'intérieur dans la direction de la largeur que les bandes induisant des plis (8, 8).

3. Couche selon l'une quelconque des revendications 1 à 2, dans laquelle le poids de base du corps absorbant (4) dans les bandes induisant des plis (8, 8) est 35 à 65 % en poids du poids de base du corps absorbant (4) dans les régions plus vers l'intérieur dans la direction de la largeur que les bandes induisant des plis (8, 8).

4. Couche selon l'une quelconque des revendications 1 à 3, dans laquelle le poids de base des fibres hydrophiles dans les bandes induisant des plis (8, 8) est 45 à 80 % en poids du poids de base des fibres hydrophiles dans les régions plus vers l'intérieur dans la direction de la largeur que les bandes induisant des plis (8, 8), et le poids de base du polymère superabsorbant dans les bandes induisant des plis (8, 8) est 25 à 50 % en poids du poids de base du polymère superabsorbant dans les régions plus vers l'intérieur dans la direction de la largeur que les bandes induisant des plis (8, 8).

5. Couche selon l'une quelconque des revendications 1 à 4, dans laquelle le poids de base du corps absorbant (4) dans les régions plus à l'extérieur dans la direction de la largeur que les bandes induisant des plis (8, 8) est 50 à 80 % en poids du poids de base du corps absorbant (4) dans les régions plus à l'intérieur dans la direction de la largeur que les bandes induisant des plis (8, 8).

# FIG. 1

FRONT

LEFT ← → RIGHT

REAR

FIG. 2

# FIG. 3

# FIG. 4

# FIG. 5

# FIG. 6

# FIG. 7

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006141761 A
- EP 0769284 A1
- WO 0032145 A1
- US 2005124951 A1
- WO 2012073499 A1